# EUROPEAN PATENT APPLICATION

(11) **EP 4 483 867 A1**
(43) Date of publication of application: **01.01.2025**
(21) Application number: 23775073.2
(22) Date of filing: 24.03.2023
(51) Int. Cl.: A61K 31/192, A61K 47/02, A61K 47/18, A61P 27/02

(54) **AQUEOUS COMPOSITION**

(30) Priority: 25.03.2022 JP 2022050275
(71) Applicant: Tsubota Laboratory, Inc., Tokyo 1608582 (JP)
(72) Inventor: ORITANI Rika, Osaka-shi, Osaka 544-8666 (JP); KITANI Chise, Osaka-shi, Osaka 544-8666 (JP); NISHIMOTO Akinori, Osaka-shi, Osaka 544-8666 (JP)
(74) Representative: Mewburn Ellis LLP
(86) International application number: PCT/JP2023/011739
(87) International publication number: WO 2023/182480

(57) **Abstract**

A first present invention relates to an aqueous composition including: (A) 4-phenylbutyric acid or esters thereof, or pharmacologically acceptable salts thereof, in which the aqueous composition is stored in a container of which a portion in contact with the aqueous composition is partly or wholly made of a resin containing a polyolefin. A second present invention relates to an aqueous composition including: (A) 4-phenylbutyric acid or esters thereof, or pharmacologically acceptable salts thereof; and (B) a chelating agent.

## Description

### Technical Field

Hereinafter, a first present invention and a second present invention will be described sequentially. The first present invention relates to an aqueous composition.

### Background Art

Sodium 4-phenylbutyrate is known to be metabolized in the body to phenylacetic acid, which is bound to glutamic acid and excreted in urine, and is used as a therapeutic agent for urea cycle abnormalities (Non-Patent Literature 1). Recently, it has been reported that sodium 4-phenylbutyrate is useful for the prevention or treatment of ocular diseases such as myopia and presbyopia (for example, Patent Literature 1 and 2).

### Citation List

### Patent Literature

[Patent Literature 1] PCT International Publication No. WO2018/164113
[Patent Literature 2] PCT International Publication No. WO2020/129965

### Non-Patent Literature

[Non-Patent Literature 1] Buphenyl (registered trademark) tablets 500 mg, Buphenyl (registered trademark) granules 94%, Package insert

### Summary of Invention

### Technical Problem

Therapeutic agents for urea cycle abnormalities with sodium 4-phenylbutyrate as an active component are commercially available in tablets and granules suitable for oral administration. On the other hand, no findings on the behavior of aqueous compositions such as eye drops containing sodium 4-phenylbutyrate when the aqueous compositions are stored in resin containers have been reported. The present inventors have found a new problem that when aqueous compositions containing sodium 4-phenylbutyrate are stored in specific resin containers, elution of impurities from the resin containers is accelerated. An object of a first present invention is to provide a novel aqueous composition which contains 4-phenylbutyric acid or a derivative thereof and is stored in a resin container and suppresses elution of impurities from the resin container.

### Solution to Problem

As a result of extensive studies to solve the above-described problem, the present inventors have found that by storing an aqueous composition containing sodium 4-phenylbutyrate in a container made of a resin containing a polyolefin, elution of impurities from the resin can be suppressed. A first present invention is based on this finding and provides each of the following inventions.
[1] An aqueous composition comprising: (A) 4-phenylbutyric acid or esters thereof, or pharmacologically acceptable salts thereof, wherein the aqueous composition is stored in a container of which a portion in contact with the aqueous composition is partly or wholly made of a resin containing a polyolefin.
[2] The aqueous composition according to [1], further comprising: (B) a buffer agent.
[3] The aqueous composition according to [1] or [2], further comprising: (C) a chelating agent.
[4] The aqueous composition according to any one of claims [1] to [3] which has a pH of 6.0 to 9.0.

### Advantageous Effects of Invention

According to the first present invention, it is possible to provide an aqueous composition which contains 4-phenylbutyric acid or a derivative thereof, is stored in a resin container, and suppresses elution of impurities from the resin container.

### Description of Embodiments

Hereinafter, an embodiment of the first present invention will be described in detail. However, the first present invention is not limited to the following embodiment.

The aqueous composition according to the present embodiment contains (A) 4-phenylbutyric acid or esters thereof, or pharmacologically acceptable salts thereof (also referred to as "component (A)").

### [Component (A)]

4-Phenylbutyric acid is also referred to as 4-PBA and is a well-known compound represented by the following formula.

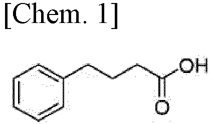

Examples of esters of 4-phenylbutyric acid include esters formed through dehydration condensation of a carboxyl group of 4-phenylbutyric acid with a C1-6 monohydric alcohol. Specific examples thereof include methyl esters, ethyl esters, n-propyl esters, isopropyl esters, n-butyl esters, isobutyl esters, sec-butyl esters, tert-butyl esters, n-pentyl esters, and n-hexyl esters. Among these, methyl esters, ethyl esters, n-propyl esters, and isopropyl esters are preferable.

Salts of 4-phenylbutyric acid and salts of esters of 4-phenylbutyric acid are not particularly limited as long as they are pharmaceutically acceptable. Specific examples thereof include metal salts such as sodium salts, potassium salts, calcium salts, and magnesium salts; inorganic salts such as ammonium salts; and organic amine salts such as triethylamine salts and guanidine salts. Among these, sodium salts and potassium salts are preferable, and sodium salts are more preferable.

4-Phenylbutyric acid or esters thereof, or pharmacologically acceptable salts thereof may be ansolvates or solvates (for example, hydrates).

The content of the component (A) in the aqueous composition according to the present embodiment is not particularly limited and is appropriately set according to the types and contents of other formulation components, formulation forms, and the like. The content of the component (A) based on the total amount of aqueous composition according to the present embodiment is, from the viewpoint of more significantly exhibiting the effect according to the first present invention, preferably 0.01 to 6 w/v%, more preferably 0.025 to 5 w/v%, still more preferably 0.05 to 4 w/v%, and particularly preferably 0.1 to 3 w/v%.

### [Component (B)]

It is preferable that the aqueous composition according to the present embodiment further contain (B) a buffer agent (also simply referred to as "component (B)"). The effect according to the first present invention is more significantly exhibited when the aqueous composition further contains the component (B). Buffer agents include inorganic buffer agents and organic buffer agents and are not particularly limited as long as they are pharmaceutically, pharmacologically (pharmaceutically), or physiologically acceptable.

Inorganic buffer agents are buffer agents derived from inorganic acids. Examples of inorganic buffer agents include boric acid buffer agents, phosphoric acid buffer agents, and carbonic acid buffer agents.

Examples of boric acid buffer agents include boric acid or salts thereof (such as alkali metal borates and alkaline earth metal borates). Examples of phosphoric acid buffer agents include phosphoric acid or salts thereof (such as alkali metal phosphates and alkaline earth metal phosphates). Examples of carbonic acid buffer agents include carbonic acid or salts thereof (such as alkali metal carbonates and alkaline earth metal carbonates). In addition, a borate hydrate or a phosphate hydrate may be used as a boric acid buffer agent or a phosphoric acid buffer agent. More specific examples thereof include boric acid or salts thereof (such as sodium borate, potassium tetraborate, potassium metaborate, ammonium borate, and borax) as boric acid buffer agents; phosphoric acid or salts thereof (such as disodium hydrogen phosphate, sodium dihydrogen phosphate, potassium dihydrogen phosphate, trisodium phosphate, tripotassium phosphate, calcium monohydrogen phosphate, and calcium dihydrogen phosphate) as phosphoric acid buffer agents; and carbonic acid or salts thereof (such as sodium hydrogen carbonate, sodium carbonate, ammonium carbonate, potassium carbonate, calcium carbonate, potassium hydrogen carbonate, and magnesium carbonate) as carbonic acid buffer agents.

Organic buffer agents are buffer agents derived from organic acids or organic bases. Examples of organic buffer agents include citric acid buffer agents, acetic acid buffer agents, tris-buffer agents, epsilon aminocaproic acid buffer agents, and AMPD buffer agents.

Examples of citric acid buffer agents include citric acid or salts thereof (such as alkali metal citrates and alkaline earth metal citrates). Examples of acetic acid buffer agents include acetic acid or salts thereof (such as alkali metal acetates and alkaline earth metal acetates). In addition, a citrate hydrate or an acetate hydrate may also be used as a citric acid buffer agent or an acetic acid buffer agent. More specific examples thereof include citric acid or salts thereof (such as sodium citrate, potassium acetate, calcium citrate, sodium dihydrogen citrate, and disodium citrate) as citric acid buffer agents; and acetic acid or salts thereof (such as ammonium acetate, potassium acetate, calcium acetate, and sodium acetate) as acetic acid buffer agents. Examples of tris-buffer agents include trometamol or salts thereof (such as trometamol hydrochloride). Examples of epsilon aminocaproic acid buffer agents include epsilon aminocaproic acid or salts thereof. Examples of AMPD buffer agents include 2-amino-2-methyl-1,3-propanediol or salts thereof.

As buffer agents, from the viewpoint of more significantly exhibiting the effect according to the first present invention, boric acid buffer agents (for example, a combination of boric acid and borax), phosphoric acid buffer agents (for example, a combination of disodium hydrogen phosphate and sodium dihydrogen phosphate), and tris-buffer agents (for example, trometamol) are preferable, boric acid buffer agents are more preferable, boric acid and salts thereof are still more preferable, and a combination of boric acid and borax is still more preferable.

Commercially available buffer agents may be used. The buffer agents may be used alone or in combination of two or more thereof.

The content of the component (B) in the aqueous composition according to the present embodiment is not particularly limited and is appropriately set according to the type of the component (B), the types and contents of other formulation components, applications and formulation forms of the aqueous composition, and the like. The content of the component (B) based on the total amount of aqueous composition is, from the viewpoint of more significantly exhibiting the effect according to the first present invention, preferably 0.05 to 5.0 w/v%, more preferably 0.08 to 4.5 w/v%, still more preferably 0.1 to 4.0 w/v%, and particularly preferably 0.3 to 3.5 w/v%.

The content ratio of the component (B) to the component (A) in the aqueous composition according to the present embodiment is not particularly limited and can be appropriately set according to the types of components (A) and (B), the types and contents of other formulation components, applications and formulation forms of the aqueous composition, and the like. The content ratio of the component (B) to the component (A) based on 1 part by mass of the total content of the component (A) in the aqueous composition according to the present embodiment is, from the viewpoint of more significantly exhibiting the effect according to the first present invention, preferably 0.008 to 500 parts by mass, more preferably 0.02 to 180 parts by mass, still more preferably 0.02 to 80 parts by mass, and particularly preferably 0.1 to 35 parts by mass.

### [Component (C)]

It is preferable that the aqueous composition according to the present embodiment further contain (C) a chelating agent (also simply referred to as "component (C)"). When the aqueous composition further contains the component (C), the preservation effectiveness is synergistically exhibited in combination with the component (A). Chelating agents are not particularly limited as long as they are pharmaceutically, pharmacologically (pharmaceutically), or physiologically acceptable.

Examples of chelating agents include ethylenediamine diacetic acid (EDDA), ethylenediamine triacetic acid, ethylenediamine tetraacetic acid (edetic acid) (EDTA), N-(2-hydroxyethyl) ethylenediamine triacetic acid (HEDTA), diethylenetriamine pentaacetic acid (DTPA), gluconic acid, and salts thereof. Examples of these salts include metal salts such as sodium salts, potassium salts, calcium salts, and magnesium salts.

As chelating agents, edetic acid or salts thereof are preferable, sodium salts of edetic acid are more preferable, disodium edetate and tetrasodium edetate are still more preferable, and disodium edetate is particularly preferable.

The content of the component (C) in the aqueous composition according to the present embodiment is not particularly limited and is appropriately set according to the type of the component (C), the types and contents of other formulation components, applications and formulation forms of the aqueous composition, and the like. The content of the component (C) based on the total amount of aqueous composition is, from the viewpoint of synergistically exhibiting the preservation effectiveness in combination with the component (A), preferably 0.001 to 12 w/v%, more preferably 0.003 to 8 w/v%, still more preferably 0.006 to 4 w/v%, and particularly preferably 0.01 to 2 w/v%.

The content ratio of the component (C) to the component (A) in the aqueous composition according to the present embodiment is not particularly limited and can be appropriately set according to the types of components (A) and (C), the types and contents of other formulation components, applications and formulation forms of the aqueous composition, and the like. The content ratio of the component (C) to the component (A) based on 1 part by mass of the total content of the component (A) in the aqueous composition according to the present embodiment is, from the viewpoint of synergistically exhibiting the preservation effectiveness in combination with the component (A), preferably 0.0002 to 1,200 parts by mass, more preferably 0.0006 to 320 parts by mass, still more preferably 0.0015 to 80 parts by mass, and particularly preferably 0.003 to 20 parts by mass.

The aqueous composition according to the present embodiment may contain a suitable amount of a combination of components selected from various pharmacologically active components and physiologically active components in addition to the above-described components within the scope not impairing the effect of the first present invention. The components are not particularly limited, and examples thereof include antiallergic agents, antihistamines, anti-inflammatory agents, steroids, decongestants, ocular muscle regulators, vitamins, amino acids, and astringents.

For the aqueous composition according to the present embodiment, various additives can be appropriately selected in accordance with usual methods according to applications and formulation forms thereof within the scope not impairing the effect of the first present invention, and one kind or two or more kinds of the additives may be incorporated in appropriate amounts in combination. Examples of such additives include carriers, pH adjusters, surfactants, flavoring agents or refreshing agents, thickeners, stabilizers, preservatives, and isotonic agents.

The pH of the aqueous composition according to the present embodiment is not particularly limited as long as it is within a pharmaceutically, pharmaceutically (pharmacologically), and physiologically acceptable range. From the viewpoint of more significantly exhibiting the effect according to the first present invention, the pH of the aqueous composition is preferably 9.0 or less, more preferably 8.5 or less, and still more preferably 8.0 or less. In addition, from the viewpoint of further improving stability of 4-phenylbutyric acid or esters thereof, or pharmacologically acceptable salts thereof, the pH of the aqueous composition is preferably 6.0 or more, more preferably 6.5 or more, and still more preferably 7.0 or more.

The aqueous composition according to the present embodiment can be adjusted to an osmotic pressure ratio within a range acceptable for living bodies as necessary. The appropriate osmotic pressure ratio can be appropriately set according to, for example, applications, formulation forms, and methods of use of the aqueous composition, and can be set to, for example, 0.4 to 5.0. The osmotic pressure ratio is a ratio of the osmotic pressure of a sample to 286 mOsm (the osmotic pressure of a 0.9 w/v% sodium chloride aqueous solution) based on the Japanese Pharmacopoeia, 18th Edition, and the osmotic pressure can be measured with reference to an osmotic pressure measurement method (freezing point depression method) described in the Japanese Pharmacopoeia. A standard solution for osmotic pressure ratio measurement (0.9 w/v% sodium chloride aqueous solution) can be prepared by drying sodium chloride (standard reagent of the Japanese Pharmacopoeia) at 500°C to 650°C for 40 to 50 minutes and then allowing it to cool in a desiccator (silica gel), and accurately weighing 0.900 g thereof and dissolving it in purified water to prepare exactly 100 mL, or a commercially available standard solution for osmotic pressure ratio measurement (0.9 w/v% sodium chloride aqueous solution) can be used.

The viscosity of the aqueous composition according to the present embodiment is not limited as long as it is within a pharmaceutically, pharmaceutically (pharmacologically), and physiologically acceptable range. As for the viscosity of the aqueous composition according to the present embodiment, the viscosity at 20°C measured using a rotational viscometer (TV-20 type viscometer manufactured by Toki Sangyo Co., Ltd., rotor; 1°34'× R24) may be 1 to 10,000 mPa·s.

The aqueous composition according to the present embodiment can be prepared, for example, by adding desired amounts of the component (A) and other components as necessary thereto and mixing them together. Specifically, for example, it can be prepared by dissolving or suspending the above-described components in purified water and sterilizing them through filtration sterilization or the like.

When the aqueous composition according to the present embodiment is an ophthalmic composition, it can be used as eye drops (also referred to as ophthalmic solutions or ophthalmic drugs, including eye drops that can be instilled while wearing contact lenses), artificial tears, eye wash (also referred to as eyewash solutions or collyrium, including eye wash that can be used to wash the eyes while wearing contact lenses). "Contact lenses" include hard contact lenses and soft contact lenses (including both ionic and nonionic ones and including both silicone hydrogel contact lenses and non-silicone hydrogel contact lenses).

Since the aqueous composition according to the present embodiment contains sodium 4-phenylbutyrate as an active component, it can be suitably used as a prophylactic agent, inhibitor, or therapeutic agent for myopia. In addition, since the aqueous composition according to the present embodiment contains sodium 4-phenylbutyrate as an active component, it can also be suitably used as a prophylactic agent, inhibitor, or therapeutic agent for presbyopia.

The aqueous composition according to the present embodiment is preferably an ophthalmic composition and more preferably eye drops (including eye drops that can be instilled while wearing contact lenses) because the effect according to the first present invention can be more significantly exhibited. When the aqueous composition according to the present embodiment is eye drops, the usage and dosage are not particularly limited as long as these exhibit an effect and cause few side effects. For example, for adults (15 years of age or older) and children of 7 years of age or older, a method of instilling 1 to 3 drops, 1 to 2 drops, or 2 to 3 drops per dose, 1 to 4 times or 5 or 6 times a day can be exemplified.

### [Container]

The aqueous composition according to the present embodiment is provided in a container of which a portion in contact with the aqueous composition is partly or wholly made of a resin (hereinafter also referred to as "resin according to the present embodiment") containing a polyolefin. Examples of resins containing a polyolefin include resins containing polyethylene (PE), polypropylene (PP), ethylene-propylene copolymers, ethylene-vinyl acetate copolymers, polymethylpentene, cyclic olefin copolymers (COP), cyclic olefin copolymers (COC), and a combination thereof.

Examples of polyethylene include high-density polyethylene (HDPE), low-density polyethylene (LDPE), and linear low-density polyethylene (LLDPE).

Cyclic olefin polymers are not limited as long as these contain polymers obtained by copolymerizing one single kind of cyclic olefins, polymers obtained by copolymerizing two or more kinds of cyclic olefins, or hydrogenated products thereof. Cyclic olefin polymers preferably contain ring-opened polymers of cyclic olefins or hydrogenated products thereof. In addition, cyclic olefin polymers preferably contain non-crystalline polymers.

Cyclic olefin copolymers are not particularly limited as long as these contain polymers obtained by copolymerizing cyclic olefins with acyclic olefins or hydrogenated products thereof.

Examples of cyclic olefins include monocyclic or polycyclic cycloalkanes having a vinyl group, monocyclic or polycyclic cycloalkenes, and derivatives thereof. The cyclic olefins are preferably norbornene, tetracyclododecene, and derivatives thereof. Examples of acyclic olefins include α-olefins such as ethylene, propylene, 1-butene, 1-pentene, and 1-hexene.

As cyclic olefin polymers, those containing polymers of cyclic olefins having a norbornene skeleton or hydrogenated products thereof are preferable from the viewpoint of more significantly exhibiting the effect according to the first present invention. As the cyclic olefin copolymers, polymers obtained by copolymerizing norbornene with ethylene are preferable from the viewpoint of more significantly exhibiting the effect according to the first present invention. Polymers obtained by copolymerizing cyclic olefins with acyclic olefins may contain other monomers as components of the polymers.

As the resin according to the present embodiment, resins containing at least one selected from the group consisting of polyethylene (PE), polypropylene (PP), and cyclic olefin copolymers (COC) are preferable, and resins containing only polyethylene, resins containing only polypropylene, and resins containing cyclic olefin copolymers and polyethylene are more preferable.

When the resin according to the present embodiment is a resin containing a cyclic olefin copolymer and polyethylene, the content ratio of a cyclic olefin copolymer to polyethylene in the resin may be, for example, 50:50 to 95:5, 55:45 to 90:10, or 60:40 to 85:15.

The resin according to the present embodiment may contain, for example, other polymers such as polycarbonates, (meth)acrylic acid polymers, polystyrene (PS), polyethylene naphthalate (PEN), polybutylene terephthalate (PBT), polyethylene terephthalate (PET), and polyarylate. In addition, the resin according to the present embodiment may contain additives such as a stabilizer, a modifier, a coloring agent, an ultraviolet absorber, a metal oxide, an oxygen absorber, an antibacterial agent, a plasticizer, and a glass fiber.

As the type of container, a container commonly used in the ophthalmic field may be used, and specific examples thereof include an eye drop container. Examples of portions of the container in contact with the aqueous composition include an inner stopper, a perforated inner stopper, and the inner surface of the container (the innermost layer if the container has a structure consisting of a plurality of layers).

The container of which a portion in contact with the aqueous composition is partly or wholly made of a resin containing polyolefin. For example, in a case of a container with a perforated inner stopper (nozzle), only the perforated inner stopper portion may be made of the resin according to the present embodiment, a storage portion or the like other than the perforated inner stopper may be made of the resin according to the present embodiment, or the entire container may be made of the resin according to the present embodiment.

Although it is sufficient as long as the portion of the container in contact with the aqueous composition is partly made of the resin according to the present embodiment, it is preferable that the portion in contact with the aqueous composition be wholly made of the resin according to the present embodiment from the viewpoint of more significantly exhibiting the effect according to the first present invention. When the container is partly made of the resin according to the present embodiment, the type of resin forming the other portion is not particularly limited, and examples thereof include polyethylene terephthalate (PET), polybutylene terephthalate (PBT), polyethylene naphthalate (PEN), polystyrene (PS), polyarylate (PAR), polycarbonate (PC), polyimide (PI), acrylonitrile-butadiene-styrene (ABS), copolymers of monomers constituting these, and mixtures of two or more thereof.

The shape and volume of the container are not particularly limited and may be appropriately set according to the application. In addition, the container may be a container (multi-dose type container) in which a multiple-use aqueous composition is stored, or may be a container (unit-dose type container) in which a single-use aqueous composition is stored.

When the container is a multi-dose type container, the volume may be, for example, 1.5 to 7.5 mL, 2 to 6 mL, or 2.5 to 5.0 mL. In addition, when the container is a unit-dose type container, the volume may be 0.1 to 1.0 mL, 0.2 to 0.9 mL, or 0.3 to 0.8 mL.

The aqueous composition according to the present embodiment can also be provided as a packed aqueous composition. The first present invention can also be considered as a pharmaceutical product (ophthalmic product such as eye drops) obtained by storing the aqueous composition of the first present invention in a container.

### [Example of first present invention]

Hereinafter, the first present invention will be specifically described based on test examples, but the first present invention is not limited to these. In addition, unless otherwise specified, the units for each component in tables are w/v%.

### [Test Example 1: Stability test in multi-dose type container]

### <Preparation of formulations>

Each aqueous composition (formulations 1-0 to 1-4) shown in Table 1-1 was prepared through a usual method to fill 5-mL volume multi-dose type containers made of polyethylene (PE), polypropylene (PP), and polyethylene terephthalate (PET) by 5 mL each.

### <Stability of sodium 4-phenylbutyrate>

Liquid chromatography was used to quantify sodium 4-phenylbutyrate. 10 µL of 0.05 w/v% sodium 4-phenylbutyrate solution was used as a standard solution, and 10 µL of a formulation diluted 10 times with a mobile phase was used as a sample solution. The standard solution and the sample solution were each placed on a reverse-phase column (YMC-Pack ODS-A (I.D. 4.6 mm × 150 mm, 5 µm) manufactured by YMC CO., LTD.) maintained at 30°C, and eluted with a 0.2% formic acid solution-acetonitrile mixture as a mobile phase. Detection was performed with an ultraviolet absorptiometer (measurement wavelength: 260 nm). Thereafter, the peak area of sodium 4-phenylbutyrate was measured for the standard solution and the sample solution, and the concentration of sodium 4-phenylbutyrate was determined for each formulation immediately after preparation and after storage at 60°C for 1 week using Equation 1 below. Table 1-2 shows results when the concentration immediately after preparation is set to 100. Concentration of sodium 4-phenylbutyrate (w/v%) = 0.05 × (peak area of 4-phenylbutyrate in sample solution/peak area of 4-phenylbutyrate in standard solution) × 10

### <Measurement of total amount of impurities>

The total amount of impurities was measured using liquid chromatography. 50 µL of a formulation diluted 100 times with purified water was used as a standard solution, and 50 µL of a formulation was used as a sample solution. The standard solution and the sample solution were each placed on a reverse-phase column (Inertsil Ph-3 HP (I.D. 4.6 mm × 250 mm, 3 µm) manufactured by GL Sciences Inc.) maintained at 45°C, and eluted using a gradient program using water/acetic acid (99:1) as a mobile phase A and acetonitrile/acetic acid (99:1) as a mobile phase B. Detection was performed with an ultraviolet absorptiometer (measurement wavelength: 254 nm). Thereafter, the peak area of sodium 4-phenylbutyrate was measured for the standard solution, the peak area of each component other than sodium 4-phenylbutyrate was measured for the sample solution, individual ratio of impurities to sodium 4-phenylbutyrate for each formulation immediately after preparation and after storage at 60°C for 1 week was calculated using Equation 2, and the sum of the ratios was obtained. The area was measured up to approximately twice the retention time of sodium 4-phenylbutyrate. In addition, the formulation 1-0 was used as a base agent. Table 1-3 shows results when the total amount of impurities immediately after preparation is set to 1. Individual ratio of impurities to 4-phenylbutyrate = (peak area of components other than 4-phenylbutyrate - peak area of components other than 4-phenylbutyrate in base agent)/peak area of 4-phenylbutyrate

**[Table 1-1]**

| Component name | Formulation 1-0 | Formulation 1-1 | Formulation 1-2 | Formulation 1-3 | Formulation 1-3' | Formulation 1-3" | Formulation 1-4 |
|---|---|---|---|---|---|---|---|
| Sodium 4-phenylbutyrate | 0 | 0.5 | 0.5 | 0.5 | 0.5 | 0.5 | 0.5 |
| Boric acid | 1 | 1 | 0 | 1 | 1 | 1 | 1 |
| Borax | 0.15 | 0.15 | 0 | 0.15 | 0.15 | 0.15 | 0.15 |
| Sodium dihydrogen phosphate | 0 | 0 | 0.05 | 0 | 0 | 0 | 0 |
| Disodium hydrogen phosphate | 0 | 0 | 0.7 | 0 | 0 | 0 | 0 |
| Disodium edetate | 0.03 | 0.03 | 0.03 | 0.03 | 0.03 | 0.03 | 0.03 |
| Hydrochloric acid/sodium hydroxide | Appropriate amount | Appropriate amount | Appropriate amount | Appropriate amount | Appropriate amount | Appropriate amount | Appropriate amount |
| Purified water | Residual amount | Residual amount | Residual amount | Residual amount | Residual amount | Residual amount | Residual amount |
| Total | 100 mL | 100 mL | 100 mL | 100 mL | 100 mL | 100 mL | 100 mL |
| pH | 7.5 | 7.5 | 7.5 | 5 | 6 | 8.5 | 10 |

**[Table 1-2]**

| | Immediately after preparation | 60°C, after 1 week | | |
|---|---|---|---|---|
| | | PE | PP | PET |
| Formulation 1-1 | 100 | 100.1 | 100.8 | 101.0 |
| Formulation 1-2 | 100 | 99.7 | 99.4 | 98.8 |
| Formulation 1-3 | 100 | 93.4 | 91.8 | 96.3 |
| Formulation 1-3' | 100 | 99.2 | 97.1 | 100.1 |
| Formulation 1-3" | 100 | 100.5 | 100.7 | 99.6 |
| Formulation 1-4 | 100 | 100.4 | 99.4 | 100.1 |

**[Table 1-3]**

| | Immediately after preparation | 60°C, after 1 week | | |
|---|---|---|---|---|
| | | PE | PP | PET |
| [Formulation 1-1]-[Formulation 1-0] | 1 | 3.5 | 2.5 | 13 |
| [Formulation 1-21-[Formulation 1-0] | 1 | 7 | 4 | 29 |
| [Formulation 1-31-[Formulation 1-0] | 1 | 13 | 4 | 54 |
| [Formulation 1-3"]-[Formulation 1-0] | 1 | 7.4 | 5.4 | 38.2 |
| [Formulation 1-4]-[Formulation 1-0] | 1 | 0.98 | 0.91 | 1.4 |

Table 1-2 shows that there was no difference in stability of sodium 4-phenylbutyrate itself among the container resins. On the other hand, it was confirmed from Table 1-3 that the generation of impurities in the formulations stored in the polyethylene resin and the polypropylene resin is suppressed compared to the formulation stored in the polyethylene terephthalate resin.

### [Test Example 2: Stability test in unit-dose type container]

### <Preparation of formulations>

An aqueous composition (formulation 2-1) shown in Table 1-4 was prepared through a usual method to fill a 0.5-mL volume polyethylene (PE) unit-dose type container and a unit-dose type resin container made of a mixture of a cyclic olefin copolymer (COC) and polyethylene (PE) by 0.5 mL each.

### <Stability of sodium 4-phenylbutyrate>

Liquid chromatography was used to quantify sodium 4-phenylbutyrate. 20 µL of 0.02 w/v% sodium 4-phenylbutyrate solution was used as a standard solution, and 20 µL of a formulation 2-1 diluted 10 times with a mobile phase was used as a sample solution. The standard solution and the sample solution were each placed on a reverse-phase column (YMC-Pack ODS-A (I.D. 4.6 mm × 150 mm, 5 µm) manufactured by YMC CO., LTD.) maintained at 30°C, and eluted with a 0.2% formic acid solution-acetonitrile mixture as a mobile phase. Detection was performed with an ultraviolet absorptiometer (measurement wavelength: 260 nm). Thereafter, the peak area of sodium 4-phenylbutyrate was measured for the standard solution and the sample solution, and the concentration of sodium 4-phenylbutyrate was determined for the formulation 2-1 immediately after preparation and after storage at 40°C for 1 month using Equation 3 below. Table 1-5 shows results when the concentration immediately after preparation is set to 100. Concentration of sodium 4-phenylbutyrate (w/v%) = 0.02 × (peak area of 4-phenylbutyrate in sample solution/peak area of 4-phenylbutyrate in standard solution) × 10

### <Measurement of total amount of impurities>

The total amount of impurities was measured using liquid chromatography. 80 µL of a formulation diluted 100 times with purified water was used as a standard solution, and 80 µL of the formulation was used as a sample solution. The standard solution and the sample solution were each placed on a reverse-phase column (Inertsil Ph-3 (I.D. 4.6 mm × 250 mm, 5 µm) manufactured by GL Sciences Inc.) maintained at 40°C, and eluted using a gradient program using water/acetic acid (99:1) as a mobile phase A and acetonitrile/acetic acid (99:1) as a mobile phase B. Detection was performed with an ultraviolet absorptiometer (measurement wavelength: 254 nm). Thereafter, the peak area of sodium 4-phenylbutyrate was measured for the standard solution, the peak area of each component other than sodium 4-phenylbutyrate was measured for the sample solution, individual ratio of impurities to sodium 4-phenylbutyrate for the formulation 2-1 immediately after preparation and after storage at 40°C for 1 month was calculated using Equation 4, and the sum of the ratios was obtained. The area was measured up to approximately twice the retention time of sodium 4-phenylbutyrate. Table 1-6 shows results when the total amount of impurities immediately after preparation is set to 1. Ratio of individual impurities to 4-phenylbutyrate = peak area of components other than 4-phenylbutyrate/peak area of 4-phenylbutyrate

**[Table 1-4]**

| | Formulation 2-1 |
|---|---|
| Sodium 4-phenylbutyrate | 0.1 |
| Boric acid | 1 |
| Borax | 0.07 |
| Sodium chloride | 0.3 |
| Potassium chloride | 0.16 |
| Hydrochloric acid/sodium hydroxide | Appropriate amount |
| Purified water | Residual amount |
| Total | 100 mL |
| pH | 7.5 |

**[Table 1-5]**

| | Immediately after preparation | 40°C, after 1 month | |
|---|---|---|---|
| | | PE | COC/PE |
| [Formulation 2-1] | 100 | 99.6 | 99.5 |

**[Table 1-6]**

| | Immediately after preparation | 40°C, after 1 month | |
|---|---|---|---|
| | | PE | COC/PE |
| [Formulation 2-1] | 1 | 5.5 | 5.7 |

Table 1-5 shows that there was no difference in stability of sodium 4-phenylbutyrate itself among the container resins. In addition, it was confirmed from Table 1-6 that the generation of impurities is suppressed in both formulations stored in the polyethylene resin and the resin containing a cyclic olefin copolymer and polyethylene.

This concludes the description of the first present invention, followed by description of the second present invention.

### [Second present invention]

The second present invention relates to an aqueous composition.

### [Background art]

Sodium 4-phenylbutyrate is known to be metabolized in the body to phenylacetic acid, which is bound to glutamic acid and excreted in urine, and is used as a therapeutic agent for urea cycle abnormalities (Non-Patent Literature 1). Recently, it has been reported that sodium 4-phenylbutyrate is useful for the prevention or treatment of ocular diseases such as myopia and presbyopia (for example, Patent Literature 1 and 2).

### [Citation list]

### [Patent literature]

[Patent Literature 1] PCT International Publication No. WO2018/164113
[Patent Literature 2] PCT International Publication No. WO2020/129965

### [Non-patent literature]

[Non-Patent Literature 1] Buphenyl (registered trademark) tablets 500 mg, Buphenyl (registered trademark) granules 94%, Package insert

### [Summary of second present invention]

### [Technical Problem to be solved by second present invention]

Therapeutic agents for urea cycle abnormalities with sodium 4-phenylbutyrate as an active component are commercially available in tablets and granules suitable for oral administration. On the other hand, while a certain level of preservative effectiveness is required for aqueous compositions such as eye drops, no findings on the preservation effectiveness of solutions containing sodium 4-phenylbutyrate have been reported. An object of the second present invention is to provide an aqueous composition which contains sodium 4-phenylbutyrate but has excellent preservation effectiveness.

### [Solution to Problem by second present invention]

The present inventors have conducted extensive studies to solve the above-described problem, and as a result, they have found that the preservation effectiveness of an aqueous composition containing sodium 4-phenylbutyrate is unexpectedly and synergistically enhanced by formulating disodium edetate which is a chelating agent in the aqueous composition. A second present invention is based on this finding and provides each of the following inventions.
[1] An aqueous composition comprising: (A) 4-phenylbutyric acid or esters thereof, or pharmacologically acceptable salts thereof; and (B) a chelating agent.
[2] The aqueous composition according to [1], wherein the content of the component (B) is 0.0002 to 1,200 parts by mass based on 1 part by mass of the total content of the component (A).
[3] The aqueous composition according to [1] or [2], further comprising: (C) a buffer agent.
[4] The aqueous composition according to any one of [1] to [3] which has a pH of 5.0 to 9.0.

### [Effect of second present invention]

According to the second present invention, it is possible to provide an aqueous composition which contains sodium 4-phenylbutyrate but has excellent preservation effectiveness.

### [Description of Embodiment of second present invention]

Hereinafter, an embodiment of the second present invention will be described in detail. However, the second present invention is not limited to the following embodiment.

The aqueous composition according to the present embodiment contains (A) 4-phenylbutyric acid or esters thereof, or pharmacologically acceptable salts thereof (also referred to as "component (A)").

### [Component (A)]

4-Phenylbutyric acid is also referred to as 4-PBA and is a well known compound represented by the following formula.

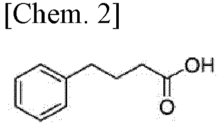

Examples of esters of 4-phenylbutyric acid include esters formed through dehydration condensation of a carboxyl group of 4-phenylbutyric acid with a C1-6 monohydric alcohol. Specific examples thereof include methyl esters, ethyl esters, n-propyl esters, isopropyl esters, n-butyl esters, isobutyl esters, sec-butyl esters, tert-butyl esters, n-pentyl esters, and n-hexyl esters. Among these, methyl esters, ethyl esters, n-propyl esters, and isopropyl esters are preferable.

Salts of 4-phenylbutyric acid and salts of esters of 4-phenylbutyric acid are not particularly limited as long as they are pharmaceutically acceptable. Specific examples thereof include metal salts such as sodium salts, potassium salts, calcium salts, and magnesium salts; inorganic salts such as ammonium salts; and organic amine salts such as triethylamine salts and guanidine salts. Among these, sodium salts and potassium salts are preferable, and sodium salts are more preferable.

4-Phenylbutyric acid or esters thereof, or pharmacologically acceptable salts thereof may be ansolvates or solvates (for example, hydrates).

The content of the component (A) in the aqueous composition according to the present embodiment is not particularly limited and is appropriately set according to the types and contents of other formulation components, formulation forms, and the like. The content of the component (A) based on the total amount of aqueous composition according to the present embodiment is, from the viewpoint of more significantly exhibiting the effect according to the second present invention, preferably 0.01 to 6 w/v%, more preferably 0.025 to 5 w/v%, still more preferably 0.05 to 4 w/v%, and particularly preferably 0.1 to 3 w/v%.

### [Component (B)]

The aqueous composition according to the present embodiment further contains (B) a chelating agent (also simply referred to as "component (B)"). Chelating agents are not particularly limited as long as they are pharmaceutically, pharmacologically (pharmaceutically), or physiologically acceptable.

Examples of chelating agents include ethylenediamine diacetic acid (EDDA), ethylenediamine triacetic acid, ethylenediamine tetraacetic acid (edetic acid) (EDTA), N-(2-hydroxyethyl) ethylenediamine triacetic acid (HEDTA), diethylenetriamine pentaacetic acid (DTPA), gluconic acid, and salts thereof. Examples of these salts include metal salts such as sodium salts, potassium salts, calcium salts, and magnesium salts.

As chelating agents, edetic acid or salts thereof are preferable, sodium salts of edetic acid are more preferable, disodium edetate and tetrasodium edetate are still more preferable, and disodium edetate is particularly preferable.

The content of the component (B) in the aqueous composition according to the present embodiment is not particularly limited and is appropriately set according to the type of the component (B), the types and contents of other formulation components, applications and formulation forms of the aqueous composition, and the like. The content of the component (B) based on the total amount of aqueous composition is, from the viewpoint of synergistically exhibiting the preservation effectiveness, preferably 0.001 to 12 w/v%, more preferably 0.003 to 8 w/v%, still more preferably 0.006 to 4 w/v%, and particularly preferably 0.01 to 2 w/v%.

The content ratio of the component (B) to the component (A) in the aqueous composition according to the present embodiment is not particularly limited and can be appropriately set according to the types of components (A) and (B), the types and contents of other formulation components, applications and formulation forms of the aqueous composition, and the like. The content ratio of the component (B) to the component (A) based on 1 part by mass of the total content of the component (A) in the aqueous composition according to the present embodiment is, from the viewpoint of more synergistically exhibiting the preservation effectiveness, preferably 0.0002 to 1,200 parts by mass, more preferably 0.0006 to 320 parts by mass, still more preferably 0.0015 to 80 parts by mass, and particularly preferably 0.003 to 20 parts by mass.

### [Component (C)]

It is preferable that the aqueous composition according to the present embodiment further contain (C) a buffer agent (also simply referred to as "component (C)"). The effect according to the second present invention is more significantly exhibited when the aqueous composition further contains the component (C). Buffer agents include inorganic buffer agents and organic buffer agents and are not particularly limited as long as they are pharmaceutically, pharmacologically (pharmaceutically), or physiologically acceptable.

Inorganic buffer agents are buffer agents derived from inorganic acids. Examples of inorganic buffer agents include boric acid buffer agents, phosphoric acid buffer agents, and carbonic acid buffer agents.

Examples of boric acid buffer agents include boric acid or salts thereof (such as alkali metal borates and alkaline earth metal borates). Examples of phosphoric acid buffer agents include phosphoric acid or salts thereof (such as alkali metal phosphates and alkaline earth metal phosphates). Examples of carbonic acid buffer agents include carbonic acid or salts thereof (such as alkali metal carbonates and alkaline earth metal carbonates). In addition, a borate hydrate or a phosphate hydrate may be used as a boric acid buffer agent or a phosphoric acid buffer agent. More specific examples thereof include boric acid or salts thereof (such as sodium borate, potassium tetraborate, potassium metaborate, ammonium borate, and borax) as boric acid buffer agents; phosphoric acid or salts thereof (such as disodium hydrogen phosphate, sodium dihydrogen phosphate, potassium dihydrogen phosphate, trisodium phosphate, tripotassium phosphate, calcium monohydrogen phosphate, and calcium dihydrogen phosphate) as phosphoric acid buffer agents; and carbonic acid or salts thereof (such as sodium hydrogen carbonate, sodium carbonate, ammonium carbonate, potassium carbonate, calcium carbonate, potassium hydrogen carbonate, and magnesium carbonate) as carbonic acid buffer agents.

Organic buffer agents are buffer agents derived from organic acids or organic bases. Examples of organic buffer agents include citric acid buffer agents, acetic acid buffer agents, tris-buffer agents, epsilon aminocaproic acid buffer agents, and AMPD buffer agents.

Examples of citric acid buffer agents include citric acid or salts thereof (such as alkali metal citrates and alkaline earth metal citrates). Examples of acetic acid buffer agents include acetic acid or salts thereof (such as alkali metal acetates and alkaline earth metal acetates). In addition, a citrate hydrate or an acetate hydrate may also be used as a citric acid buffer agent or an acetic acid buffer agent. More specific examples thereof include citric acid or salts thereof (such as sodium citrate, potassium acetate, calcium citrate, sodium dihydrogen citrate, and disodium citrate) as citric acid buffer agents; and acetic acid or salts thereof (such as ammonium acetate, potassium acetate, calcium acetate, and sodium acetate) as acetic acid buffer agents. Examples of tris-buffer agents include trometamol or salts thereof (such as trometamol hydrochloride). Examples of epsilon aminocaproic acid buffer agents include epsilon aminocaproic acid or salts thereof. Examples of AMPD buffer agents include 2-amino-2-methyl-1,3-propanediol or salts thereof.

As buffer agents, from the viewpoint of more significantly exhibiting the effect according to the second present invention, boric acid buffer agents (for example, a combination of boric acid and borax), phosphoric acid buffer agents (for example, a combination of disodium hydrogen phosphate and sodium dihydrogen phosphate), and tris-buffer agents (for example, trometamol) are preferable, boric acid buffer agents are more preferable, boric acid and salts thereof are still more preferable, and a combination of boric acid and borax is still more preferable.

Commercially available buffer agents may be used. The buffer agents may be used alone or in combination of two or more thereof.

The content of the component (C) in the aqueous composition according to the present embodiment is not particularly limited and is appropriately set according to the type of the component (C), the types and contents of other formulation components, applications and formulation forms of the aqueous composition, and the like. The content of the component (C) based on the total amount of aqueous composition is, from the viewpoint of more significantly exhibiting the effect according to the second present invention, preferably 0.05 to 5.0 w/v%, more preferably 0.08 to 4.5 w/v%, still more preferably 0.1 to 4.0 w/v%, and particularly preferably 0.3 to 3.5 w/v%.

The content ratio of the component (C) to the component (A) in the aqueous composition according to the present embodiment is not particularly limited and can be appropriately set according to the types of components (A) and (C), the types and contents of other formulation components, applications and formulation forms of the aqueous composition, and the like. The content ratio of the component (C) to the component (A) based on 1 part by mass of the total content of the component (A) in the aqueous composition according to the present embodiment is, from the viewpoint of more significantly exhibiting the effect according to the second present invention, preferably 0.008 to 500 parts by mass, more preferably 0.02 to 180 parts by mass, still more preferably 0.03 to 80 parts by mass, and particularly preferably 0.1 to 35 parts by mass.

The aqueous composition according to the present embodiment may contain a suitable amount of a combination of components selected from various pharmacologically active components and physiologically active components in addition to the above-described components within the scope not impairing the effect of the second present invention. The components are not particularly limited, and examples thereof include antiallergic agents, antihistamines, anti-inflammatory agents, steroids, decongestants, ocular muscle regulators, vitamins, amino acids, and astringents.

For the aqueous composition according to the present embodiment, various additives can be appropriately selected in accordance with usual methods according to applications and formulation forms thereof within the scope not impairing the effect of the second present invention, and one kind or two or more kinds of the additives may be incorporated in appropriate amounts in combination. Examples of such additives include carriers, pH adjusters, surfactants, flavoring agents or refreshing agents, thickeners, stabilizers, preservatives, and isotonic agents.

The pH of the aqueous composition according to the present embodiment is not particularly limited as long as it is within a pharmaceutically, pharmaceutically (pharmacologically), and physiologically acceptable range. From the viewpoint of more significantly exhibiting the effect according to the second present invention, the pH of the aqueous composition is preferably 9.0 or less, more preferably 8.5 or less, and still more preferably 8.0 or less. In addition, from the viewpoint of further improving stability of 4-phenylbutyric acid or esters thereof, or pharmacologically acceptable salts thereof, the pH of the aqueous composition is preferably 5.0 or more, more preferably 5.5 or more, and still more preferably 6.0 or more.

The aqueous composition according to the present embodiment can be adjusted to an osmotic pressure ratio within a range acceptable for living bodies as necessary. The appropriate osmotic pressure ratio can be appropriately set according to, for example, applications, formulation forms, and methods of use of the aqueous composition, and can be set to, for example, 0.4 to 5.0. The osmotic pressure ratio is a ratio of the osmotic pressure of a sample to 286 mOsm (the osmotic pressure of a 0.9 w/v% sodium chloride aqueous solution) based on the Japanese Pharmacopoeia, 18th Edition, and the osmotic pressure can be measured with reference to an osmotic pressure measurement method (freezing point depression method) described in the Japanese Pharmacopoeia. A standard solution for osmotic pressure ratio measurement (0.9 w/v% sodium chloride aqueous solution) can be prepared by drying sodium chloride (standard reagent of the Japanese Pharmacopoeia) at 500°C to 650°C for 40 to 50 minutes and then allowing it to cool in a desiccator (silica gel), accurately weighing 0.900 g thereof, and dissolving it in purified water to prepare exactly 100 mL, or a commercially available standard solution for osmotic pressure ratio measurement (0.9 w/v% sodium chloride aqueous solution) can be used.

The viscosity of the aqueous composition according to the present embodiment is not limited as long as it is within a pharmaceutically, pharmaceutically (pharmacologically), and physiologically acceptable range. As for the viscosity of the aqueous composition according to the present embodiment, the viscosity at 20°C measured using a rotational viscometer (TV-20 type viscometer manufactured by Toki Sangyo Co., Ltd., rotor; 1°34'×R24) may be 1 to 10,000.

The aqueous composition according to the present embodiment can be prepared, for example, by adding desired amounts of the component (A), the component (B), and other components as necessary thereto and mixing them together. Specifically, for example, it can be prepared by dissolving or suspending the above-described components in purified water and sterilizing them through filtration sterilization or the like.

When the aqueous composition according to the present embodiment is an ophthalmic composition, it can be used as eye drops (also referred to as ophthalmic solutions or ophthalmic drugs and include eye drops that can be instilled while wearing contact lenses), artificial tears, eye wash (also referred to as eyewash solutions or collyrium and include eye wash that can be used to wash the eyes while wearing contact lenses). "Contact lenses" include hard contact lenses and soft contact lenses (including both ionic and nonionic ones and including both silicone hydrogel contact lenses and non-silicone hydrogel contact lenses).

Since the aqueous composition according to the present embodiment contains sodium 4-phenylbutyrate as an active component, it can be suitably used as a prophylactic agent, inhibitor, or therapeutic agent for myopia. In addition, since the aqueous composition according to the present embodiment contains sodium 4-phenylbutyrate as an active component, it can also be suitably used as a prophylactic agent, inhibitor, or therapeutic agent for presbyopia.

The aqueous composition according to the present embodiment is preferably an ophthalmic composition and more preferably eye drops (including eye drops that can be instilled while wearing contact lenses) because the effect according to the second present invention can be more significantly exhibited. When the aqueous composition according to the present embodiment is eye drops, the usage and dosage are not particularly limited as long as these exhibit an effect and cause few side effects. For example, for adults (15 years of age or older) and children of 7 years of age or older, a method of instilling 1 to 3 drops, 1 to 2 drops, or 2 to 3 drops per dose, 1 to 4 times or 5 or 6 times a day can be exemplified.

The aqueous composition according to the present embodiment is stored and provided in any container. The container that stores the aqueous composition according to the present embodiment is not particularly limited, and may be made of, for example, glass or plastic. The container is preferably made of plastic. Examples of plastic include polyolefin resins such as polyethylene, polypropylene, cyclic olefin copolymers, and a mixture of two or more thereof, polyester resins such as polyethylene terephthalate, polybutylene terephthalate, polyethylene naphthalate, and a mixture of two or more thereof. As the material of the container, a polyolefin resin is more preferable from the viewpoint of further enhancing the effect of the second present invention. Plastic may contain, for example, other polymers such as polycarbonates, (meth)acrylic acid polymers, polystyrene (PS), and polyarylate. In addition, plastic may also contain additives such as a stabilizer, a modifier, a coloring agent, an ultraviolet absorber, a metal oxide, an oxygen absorber, an antibacterial agent, a plasticizer, and a glass fiber. In addition, elastomers such as styrene thermoplastic elastomers and styrene-butadiene thermoplastic elastomer may be used in the container that stores the aqueous composition according to the present embodiment. In addition, the container that stores the aqueous composition according to the present embodiment may be a transparent container of which the inside can be visually recognized, or may be an opaque container of which the inside is difficult to be visually recognized. The container is preferably a transparent container. The term "transparent container" includes both colorless and colored transparent containers.

A nozzle may be mounted in the container that stores the aqueous composition according to the present embodiment. The material of the nozzle is not particularly limited, and the nozzle may be made of, for example, glass or plastic. The container is preferably made of plastic. Examples of plastic include polyolefin resins such as polyethylene, polypropylene, cyclic olefin copolymers, and a mixture of two or more thereof, polyester resins such as polyethylene terephthalate, polybutylene terephthalate, polyethylene naphthalate, and a mixture of two or more thereof. As the material of the nozzle, a polyolefin resin is more preferable from the viewpoint of further enhancing the effect of the second present invention. Plastic may contain, for example, other polymers such as polycarbonates, (meth)acrylic acid polymers, polystyrene (PS), and polyarylate. In addition, plastic may also contain additives such as a stabilizer, a modifier, a coloring agent, an ultraviolet absorber, a metal oxide, an oxygen absorber, an antibacterial agent, a plasticizer, and a glass fiber. In addition, a silicone may be used in the nozzle mounted in the container that stores the aqueous composition according to the present embodiment.

The shape and volume of the container are not particularly limited and may be appropriately set according to the application. In addition, the container may be a container (multi-dose type container) in which a multiple-use aqueous composition is stored, or may be a container (unit-dose type container) in which a single-use aqueous composition is stored.

When the container is a multi-dose type container, the volume may be, for example, 1.5 to 7.5 mL, 2.0 to 6.0 mL, or 2.5 to 5.0 mL. In addition, when the container is a unit-dose type container, the volume may be 0.1 to 1.0 mL, 0.2 to 0.9 mL, or 0.3 to 0.8 mL.

The aqueous composition according to the present embodiment can also be provided as a packed aqueous composition. The second present invention can also be considered as a pharmaceutical product (ophthalmic product such as eye drops) obtained by storing the aqueous composition of the second present invention in a container.

### [Example of second present invention]

Hereinafter, the second present invention will be specifically described based on test examples, but the second present invention is not limited to these. In addition, unless otherwise specified, the units for each component in tables are w/v%.

### [Test Example 1: Preservation effectiveness test]

Each aqueous composition shown in Table 2-1 was prepared through a usual method, filtered through a 0.2 µm membrane filter, and sterilized. The units for each component in Table 2-1 are w/v%. Thereafter, preservation effectiveness tests of each aqueous composition were conducted based on the Japanese Pharmacopoeia, 18th Edition. *Pseudomonas aeruginosa* was inoculated on the surface of Soybean Casein Digest Slant Medium and cultured at 30°C to 35°C for 24 hours. Cultured bacteria were collected aseptically using a platinum loop and were made to be suspended in an appropriate amount of sterile physiological saline to prepare a bacterial suspension containing about 1×10⁷ CFU/mL. The number of viable bacteria in the bacterial suspension was separately cultured and measured. Next, 15 mL centrifuge tubes (PET) were filled with each prepared aqueous composition by 10 mL each. Each of these aqueous compositions was inoculated with the bacterial suspension so that the number of viable bacteria (final concentration) was approximately 5×10⁵ CFU/mL and well stirred to obtain samples. The samples containing the bacteria were preserved at 20°C to 25°C for 7 days. Thereafter, the samples containing the bacteria were adjusted to an appropriate concentration for counting, the bacteria were collected according to an agar streak method and cultured in Soybean Casein Digest Agar Medium at 30°C to 35°C for 2 to 3 days, and the number of colonies observed was counted to determine the number of viable bacteria. The number of viable bacteria immediately after inoculation was compared with the number of viable bacteria in the samples after 7 days of preservation, and reduction in the number of bacteria was calculated as preservation effectiveness (log reduction). The results are shown in Table 2-1.

**[Table 2-1]**

| Component name | Comparative Example 1 | Comparative Example 2 | Comparative Example 3 | Example 1 |
|---|---|---|---|---|
| Sodium 4-phenylbutyrate | 0 | 0 | 2 | 2 |
| Boric acid | 0.5 | 0.5 | 0.5 | 0.5 |
| Borax | 0.025 | 0.025 | 0.025 | 0.025 |
| Disodium edetate | 0 | 0.03 | 0 | 0.03 |
| Hydrochloric acid/sodium hydroxide | Appropriate amount | Appropriate amount | Appropriate amount | Appropriate amount |

| Purified water | Residual amount | Residual amount | Residual amount | Residual amount |
|---|---|---|---|---|
| Total | 100 mL | 100 mL | 100 mL | 100 mL |
| pH | 7.5 | 7.5 | 7.5 | 7.5 |
| Preservation effectiveness (Log reduction) | 1.3 | 1.7 | 2.9 | > 4.7 |

It was confirmed in the aqueous composition containing sodium 4-phenylbutyrate and disodium edetate that the number of inoculated bacteria was reduced to less than a detection limit and the preservation effectiveness (log reduction) value was greater than 4.7 (Example 1). In the aqueous compositions containing no sodium 4-phenylbutyrate, the preservation effectiveness (log reduction) was increased by only 0.4 due to incorporation of disodium edetate remained 0.4 (compare between Comparative Examples 1 and 2). On the other hand, in the aqueous compositions containing sodium 4-phenylbutyrate, the preservation effectiveness (log reduction) was increased by at least about 2 due to incorporation of disodium edetate (compare between Comparative Example 3 and Example 1). From the above, it was confirmed that the combination of sodium 4-phenylbutyrate and disodium edetate synergistically increases the preservation effectiveness.

## Claims

1. An aqueous composition comprising:
(A) 4-phenylbutyric acid or esters thereof, or pharmacologically acceptable salts thereof,
wherein the aqueous composition is stored in a container of which a portion in contact with the aqueous composition is partly or wholly made of a resin containing a polyolefin.

2. The aqueous composition according to claim 1, further comprising:
(B) a buffer agent.

3. The aqueous composition according to claim 1 or 2, further comprising:
(C) a chelating agent.

4. The aqueous composition according to claim 1 or 2 which has a pH of 6.0 to 9.0.

5. An aqueous composition comprising:
(A) 4-phenylbutyric acid or esters thereof, or pharmacologically acceptable salts thereof; and
(B) a chelating agent.

6. The aqueous composition according to claim 5,
wherein the content of the component (B) is 0.0002 to 1,200 parts by mass based on 1 part by mass of the total content of the component (A).

7. The aqueous composition according to claim 5 or 6, further comprising:
(C) a buffer agent.

8. The aqueous composition according to claim 5 or 6 which has a pH of 5.0 to 9.0.
